# EUROPEAN PATENT APPLICATION

(11) **EP 4 579 675 A1**
(43) Date of publication of application: **02.07.2025**
(21) Application number: 23220284.6
(22) Date of filing: 27.12.2023
(51) Int. Cl.: G16H 20/40, A61B 34/30, A61B 90/00, B25J 9/16, G16H 40/63

(54) **SURGICAL IMAGING SYSTEM AND CORRESPONDING SYSTEM, METHOD AND COMPUTER PROGRAM FOR A SURGICAL IMAGING SYSTEM**

(71) Applicant: Leica Instruments (Singapore) Pte. Ltd., Singapore 608924 (SG)
(72) Inventor: THEMELIS, George, 608924 Singapore (SG)
(74) Representative: 2SPL Patentanwälte PartG mbB

(57) **Abstract**

Examples relate to a system for a surgical imaging system, to a method for a surgical imaging system, a corresponding computer program, and to a surgical imaging system. A system (110) for a surgical imaging system (100) is configured to obtain a trigger for executing a set of documentation actions for documenting a surgical site (10) of a surgical procedure being performed with the help of the surgical imaging system, execute the set of documentation actions in response to the trigger, the set of documentation actions comprising a plurality of actions to be performed for documenting the surgical procedure, and store a result of the execution of the set of documentation actions.

## Description

### Technical field

Examples relate to a system for a surgical imaging system, to a method for a surgical imaging system, a corresponding computer program, and to a surgical imaging system.

### Background

There are various applications, where multiple images or media are captured simultaneously or sequentially, for different purposes. For example, many mobile phones offer an image capturing mode in which, when an image is captured, the device saves a short video clip spanning from a few seconds before and a few seconds after the press of the button. This creates a "live" image that adds context and movement to a photo and makes it more dynamic and, thus, easier to comprehend. Moreover, some mobile phone applications allow simultaneously saving images from multiple cameras on the phone. For example, the front and the back camera images may be captured simultaneously to prove the presence of a person in a certain location. For example, when somebody posts a picture from a concert on social media, the additional photo from the front camera proves that, indeed, this person was there and took the photo. High Dynamic Range (HDR) photography is typically performed by the sequential acquisition of multiple images with different sensitivity settings. Then the images are combined into a single HDR image.

Handheld 3D scanning devices allow the user to capture images from multiple angles. These images are then processed using software to create a 3D model of an object or space. Some optical imaging devices, such as industrial microscopes, have a function to perform an imaging sequence of multiple working distances and reprocess the images to create a 3D scan of the object. Another example is biomedical imaging, where a microscope scans a slide by capturing multiple images and recombining them to create a very high-resolution collage image of the whole slide.

Modern surgical imaging devices, such as surgical microscopes, contain many different imaging modalities. However, the documentation of surgical procedures being performed with the help of surgical imaging devices is still a largely manual process, which leads to a spurious documentation of the surgical procedure, to a high cognitive load expected from the surgeon, or both. There may be a desire for providing an improved concept for documenting surgical procedures being performed with the help of a surgical imaging device.

### Summary

This desire is addressed by the subject-matter of the independent claims.

The proposed concept is based on the finding that other systems may only partially address the needs of documenting surgical procedures being performed with a surgical microscope or other surgical imaging device. A surgical microscope is a complex instrument with multiple imaging modes, and different settings such as illumination filters, focus and zoom. Moreover, microscopes become ever more complex in terms of components and technologies used, e.g., as a robotic arm, different imaging filters, multiple light sources, and polarizers may be used. This creates many different setting combinations that could be used for imaging. While these many different setting combinations enable a surgeon to gain new insights during the surgical procedure, they also provide an overwhelming number of options to be available for documenting the surgical procedure. Thus, in some cases, a surgeon may choose less then optimal options for documenting the surgical procedure or choose to omit documenting the surgical procedure in the first place to shed the cognitive load. To facilitate the process of documenting the surgical procedure for the surgeon, the proposed concept is based on defining a set of documentation actions to be performed for documenting the surgical procedure, which are triggered and then performed without requiring intervention by the surgeon, thus removing the burden of doing the documentation from the surgeon. For example, the set of documentation actions may be triggered automatically at suitable stages of the surgical procedure, e.g., when the surgeon changes the surgical tool being used, or when a new stage of the surgical procedure is reached.

Some aspects of the present disclosure relate to a system for a surgical imaging system. The system comprises one or more processors and one or more storage devices. The system is configured to obtain a trigger for executing a set of documentation actions for documenting a surgical site of a surgical procedure being performed with the help of the surgical imaging system. The system is configured to execute the set of documentation actions in response to the trigger. The set of documentation actions comprises a plurality of actions to be performed for documenting the surgical procedure. The system is configured to store a result of the execution of the set of documentation actions. By triggering an entire set of documentation actions comprising a plurality of actions to be performed for documenting the surgical procedure, a comprehensive documentation of the surgical procedure may be reached while reducing the cognitive load on the surgeon.

As outlined above, many surgical imaging devices, such as surgical microscopes or surgical exoscopes, have different imaging modes. The set of documentation actions may comprise an action comprising recording one or more images of the surgical site using a first imaging mode and a further action comprising recording one or more images of the surgical site using a second imaging mode. By recording the surgical site using multiple imaging modes, a more comprehensive representation of the surgical site may be recorded and stored for documentation purposes.

While the surgeon will usually work on a tiny portion of the body of a patient, in some cases, it may be useful to also have a documentation of a zoomed-out view, e.g., for the purpose of determining whether bleeding exists outside the immediate surgical site. For example, the set of documentation actions may comprise an action comprising recording one or more images of the surgical site having a first field of view (e.g., a zoomed-in field of view) and a further action comprising recording one or more images of the surgical site having a second field of view (e.g., a zoomed-out field of view).

Not only the zoomed-in operation of the surgical imaging device during the surgical procedure, but also the focal plane with narrow depth of field may cause incidents to be hidden during documentation. Therefore, the set of documentation actions may comprise an action comprising recording one or more images of the surgical site having a first focal plane and a further action comprising recording one or more images of the surgical site having a second focal plane. By covering multiple focal planes, incidents or occurrences outside the focal plane being used by the surgeon may be documented as well.

In many cases, surgical procedures are performed on narrow wound tracts, in an effort to accelerate the healing process and to reduce the size of scars. However, such narrow wound tracts may partially obstruct the view on the surgical site if only one angle of observation is used. Accordingly, the set of documentation actions may comprise an action comprising recording one or more images of the surgical site having a first angle of observation and a further action comprising recording one or more images of the surgical site having a second angle of observation. By covering multiple angles of observation, incidents or occurrences may be uncovered that would be obstructed otherwise.

For example, the system may be configured to control at least one mechanical element of the surgical imaging system, such as a robotic arm, a motorized stand, or one or more mirrors, between between the recording of the one or more images of the surgical site having the first angle of observation and the recording of the one or more images of the surgical site having the second angle of observation. This way, multiple angles of observation may be captured without requiring use of multiple imaging sensors or cameras and without the surgical staff having to reposition the surgical imaging device.

Generally, it may be advantageous to choose the first and second angle of observation such that existing obstructions are overcome. For example, the system may be configured to determine one or more obstacles that at least partially block the view on the surgical site. The system may be configured to determine at least one of the first angle of observation and the second angle of observation based on the one or more obstacles. This way, incidents or occurrences may be uncovered that would be obstructed otherwise. Moreover, if it is determined that no such obstacles exist, capturing images from multiple angles of observation may be foregone, which may reduce the size of the result.

While documenting a surgical site using nearly simultaneously taken images provides a comprehensive representation of the surgical site, sequences of images (e.g., a video) may provide additional context, e.g., regarding the flow of blood, movement of tissue etc. For example, the set of documentation actions may comprise at least one action comprising recording a sequence of images of the surgical site, with the sequence of images having a pre-defined length. This way, some additional context is stored in the result, with the pre-defined length helping to make the amount of time required for collecting the documentation imagery deterministic, in the case additional still images are to be taken using different imaging characteristics, angles of observation, field of view etc.

Performing the set of documentation actions may require some time, during which the surgical imaging device is not available to the surgeon. In general, the surgeon is the one best qualified to decide when they can dispense with the use of the surgical imaging device for some time (e.g., after a stage or activity of the surgical procedure has concluded). For example, the system may be configured to obtain the trigger from a user (e.g., the surgeon) of the surgical imaging system via an input device of the surgical imaging system. This way, the surgeon can make sure that the surgical imaging system is always available for use when the surgeon needs it.

To reduce the cognitive load of the surgeon, the trigger for executing the set of documentation actions may be generated by the system itself. For example, the system may be configured to process imaging sensor data of a surgical imaging device of the surgical imaging system to determine a progress of the surgical procedure. The system may be configured to generate the trigger according to the progress of the surgical procedure. This way, the cognitive load may be reduced for the surgeon.

For example, the system may be configured to process imaging sensor data of a surgical imaging device of the surgical imaging system to determine an activity of a surgeon at a surgical site during the surgical procedure. The system may be configured to generate the trigger in response to the surgeon removing a surgical tool from the surgical site. Removing a surgical tool from the surgical site may be seen as indicator that a stage of the surgical procedure is over, which is a suitable time for documenting the surgical procedure as the surgical staff often requires some time (e.g., for preparing the surgical tools) before proceeding to the next stage of the surgical procedure.

During surgical procedures, there are some opportunities, during which multiple documentation actions can be performed without causing an impairment of the ability of the surgeon to use the surgical imaging device. For example, when a surgical imaging device changes between different imaging modalities or imaging modes, a slight delay may be introduced by the switch. The system may be configured to detect a switch between different imaging modalities or imaging modes during the surgical procedure, and to generate the trigger based on the switch between the different imaging modalities. At the switch between different imaging modalities or imaging modes, two documentation actions may be performed: a first one using the imaging modality or imaging mode being used before the switch, and a second one using the imaging modality or imaging mode being used after the switch. Moreover, during the switch, a slight delay may be introduced, which may be used for one or more further documentation actions in addition the first and second imaging modality.

In some examples, the system may be configured to generate the trigger according to a timer. By using a timer, it may be ensured that documentation is performed with a sufficient regularity.

As outlined above, executing a full set of documentation actions, including repositioning at least one mechanical element of the surgical imaging system, recording a sequence of images etc., may require some time, which may not be available at any given time during the surgical procedure. However, some documentation actions may take little time and may not impair the use of the surgical imaging device. Therefore, two different sets of documentation actions may be defined, to be used depending on whether time is available for performing the full set of documentation actions (e.g., between stages of the surgical procedure) or only time for performing documentation actions that do not impair the use of the surgical imaging device (e.g., when switching between different imaging modalities). For example, the system may be configured to obtain a first trigger and a second trigger, and to execute a first set of documentation actions in response to the first trigger and a second set of documentation actions in response to the second trigger. For example, the first set of documentation actions may take a longer time to execute than the second set of documentation actions.

For example, the first, more comprehensive set of documentation actions may be triggered manually, while the second, less comprehensive set of documentation actions (which may not or only slightly impair using the surgical imaging device) may be triggered automatically. For example, the system may be configured to obtain the first trigger from a user via an input device of the surgical imaging system, and to obtain the second trigger by generating the trigger based on at least one of a progress of the surgical procedure, an activity of a surgeon at a surgical site, a switch between imaging modalities, and a timer. This way, even if the second set of documentation actions is triggered at an inopportune time, there might not be a major impact on the work of the surgeon.

While performing multiple documentation actions is useful for having a comprehensive representation of the surgical procedure, keeping the results of the different documentation actions separate may reduce the usefulness and understandability of the result. Therefore, the system may be configured to generate a combined representation of the result of at least two different documentation actions. Such a combined representation may allow a user to better understand the documentation of the surgical procedure.

In general, surgical procedures are used to affect a certain physical outcome, like removing a tumor. To understand the progress being made towards the outcome, it may be beneficial to visualize a progress of the surgical procedure. For example, the system may be configured to, for at least one documentation action of the set of documentation actions, generate a visual representation of a difference between the results of successive executions of the documentation action. This way, a user can better grasp the progress of the surgical procedure.

In some cases, the amount of data/imagery being captured may allow for a 3D reconstruction of the surgical site. For example, the system may be configured to, using the result of the execution of the set of documentation actions, generate a three-dimensional representation of a surgical site being operated on. This may allow a user to examine the surgical procedure from different angles.

In some cases, the captured images may be combined to provide a more detailed view of the surgical site. For example, the system may be configured to, using the result of the execution of the set of documentation actions, generate a collage of a plurality of images of a surgical site being operated on. For example, the collage may have a higher resolution than individual images of the plurality of images. This collage may be used to gain a more detailed understanding of the surgical procedure.

Another aspect of the present disclosure relates to a surgical imaging system comprising a surgical imaging device and the system described above. For example, the surgical imaging device may be one of a (surgical) microscope and a (surgical) exoscope.

Another aspect of the present disclosure relates to a corresponding method for the surgical imaging system. The method comprises obtaining a trigger for executing a set of documentation actions for documenting a surgical site of a surgical procedure being performed with the help of the surgical imaging system. The method comprises executing the set of documentation actions in response to the trigger, the set of documentation actions comprising a plurality of actions to be performed for documenting the surgical procedure. The method comprises storing a result of the execution of the set of documentation actions.

Another aspect of the present disclosure relates to computer program with a program code for performing the above method when the computer program is run on a processor.

### Short description of the Figures

Some examples of apparatuses and/or methods will be described in the following by way of example only, and with reference to the accompanying figures, in which:
- Fig. 1a: shows a block diagram of an example of a system for a surgical imaging system;
- Fig. 1b: shows a schematic diagram of an example of a surgical microscope system;
- Fig. 2: shows a flow chart of a method for a surgical imaging system; and
- Fig 3: shows a schematic diagram of an example of a system comprising a surgical imaging device and a computer system.

### Detailed Description

Various examples will now be described more fully with reference to the accompanying drawings in which some examples are illustrated. In the figures, the thicknesses of lines, layers and/or regions may be exaggerated for clarity.

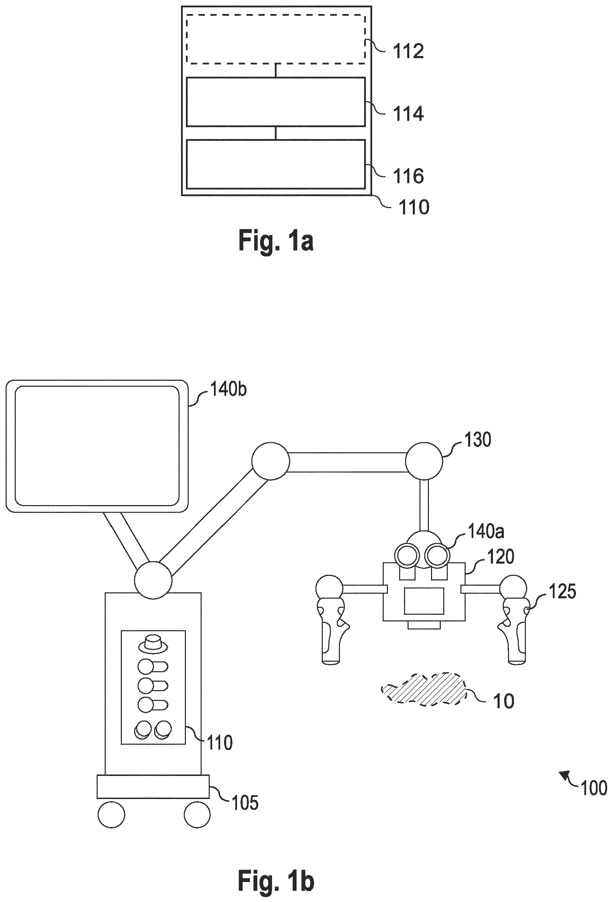

Fig. 1a shows a block diagram of an example of a system 110 for a surgical imaging system 100 (shown in Fig. 1b). The system 110 is a component of the surgical imaging system 100 and may be used to control various aspects of the surgical imaging system 100. In particular, it may be used for acquisition and/or processing of imaging sensor data by an optical imaging sensor of an imaging device 120 of the surgical imaging system or other sensor data, through various means that will be introduced in more detail in the following. In addition, the system 110 may be configured to control additional aspects of the surgical imaging system, e.g., to provide a display signal for various displays of the surgical imaging system.

In general, the system 110 may be considered to be a computer system. The system 110 comprises one or more processors 114 and one or more storage devices 116. Optionally, the system 110 further comprises one or more interfaces 112. The one or more processors 114 are coupled to the one or more storage devices 116 and to the one or more interfaces 112. In general, the functionality of the system 110 may be provided by the one or more processors 114, in conjunction with the one or more interfaces 112 (for exchanging data/information with one or more other components of the surgical imaging system 100 and outside the surgical imaging system 100, such as an optical imaging sensor of the imaging device 120 and/or a robotic arm 130 of the surgical imaging system), and with the one or more storage devices 116 (for storing information, such as machine-readable instructions of a computer program being executed by the one or more processors). In general, the functionality of the one or more processors 114 may be implemented by one or more processors 114 executing machine-readable instructions. Accordingly, any feature ascribed to the one or more processors 114 may be defined by one or more instructions of a plurality of machine-readable instructions. The system 110 may comprise the machine-readable instructions, e.g., within the one or more storage devices 116.

As outlined above, the system 110 is part of the surgical imaging system 100, which comprises various components in addition to the system 110. For example, the surgical imaging system 100 comprises the imaging device 120, and may comprise one or more additional components, such as a robotic arm 130. Fig. 1b shows a schematic diagram of an example of such a surgical imaging system 100, and in particular of a surgical microscope system 100. In the following, the surgical imaging system 100 may also be referred to as surgical microscope system 100, which is a surgical imaging system 100 that comprises a (surgical) microscope as imaging device 120. However, the proposed concept is not limited to such embodiments. The surgical imaging system 100 may be based on various (single or multiple) imaging devices, such as one or more microscopes, one or more endoscopes, one or more exoscopes (also sometimes called an extracorporeal telescope). Exoscopes are camera-based imaging systems, and in particular camera-based 3D imaging systems, which are suitable for providing images of surgical sites with high magnification and a large depth of field. Compared to microscopes, which may be used via oculars, exoscopes are only used via display modalities, such as monitor or a head-mounted display. Accordingly, the surgical imaging system may alternatively be a surgical endoscope system, or a surgical exoscope system. The following illustrations assume that the imaging device 120 is a surgical microscope for use in neurosurgery, and that the surgical imaging system 100 is a surgical microscope system 100 for use in neurosurgery.

Accordingly, the surgical imaging system or surgical microscope system 100 may comprise an imaging device, such as a microscope 120, an endoscope or an exoscope. In general, a microscope, such as the microscope 120, is an optical instrument that is suitable for examining objects that are too small to be examined by the human eye (alone). For example, a microscope may provide an optical magnification of a sample. In the present concept, the optical magnification is (also) provided for an optical imaging sensor. The microscope 120 thus comprises an optical imaging sensor, which is coupled with the system 110. The microscope 120 may further comprise one or more optical magnification components that are used to magnify a view on the sample, such as an objective (i.e., lens). For example, the surgical imaging device or microscope 120 is often also called the "optics carrier" of the surgical imaging system.

There are a variety of different types of surgical imaging devices. If the surgical imaging device is used in the medical or biological fields, the object being viewed through the surgical imaging device may be a sample of organic tissue, e.g., arranged within a petri dish or present in a part of a body of a patient. In various examples presented here, the imaging device 120 may be a microscope of a surgical microscope system, i.e., a microscope that is to be used during a surgical procedure, such as a neurosurgical procedure or an ophthalmological procedure (i.e., eye surgery). Accordingly, the object being viewed through the surgical imaging device (that is shown in the field of view of the surgical imaging device) and shown in the digital view generated by based on the imaging sensor data provided by the (optional) optical imaging sensor, may be a sample of organic tissue of a patient, and may be in particular be the surgical site that the surgeon operates on during the surgical procedure, e.g., the brain or the eye. However, the proposed concept is also suitable for other types of surgery, such as cardiac surgery.

Fig. 1b shows a schematic diagram of an example of a surgical imaging system 100, and in particular of a surgical microscope system 100 for use in neurosurgery, comprising the system 110 and a microscope 120. The surgical microscope system 100 shown in Fig. 1b comprises a number of optional components, such as a base unit 105 (comprising the system 110) with a (rolling, e.g. motorized) stand, ocular displays 140a that are arranged at the microscope 120, an auxiliary display 140b that is arranged at the base unit, the robotic arm 130 that holds the microscope 120 in place, and which is coupled to the base unit 105 and to the microscope 120. In general, these optional and non-optional components may be coupled to the system 110 which may be configured to control and/or interact with the respective components.

In the proposed concept, the system is used, to provide a digital view on a surgical site, and to document the surgical procedure. During surgical procedures, documentation is an important but difficult topic. On the one hand, the documentation of the surgical procedure is useful for a post-surgical analysis of the surgical procedure, and for determining, after the surgery, whether an infection or bleeding could have been prevented. On the other hand, proper documentation requires effort on part of the surgeon - for determining, which imaging mode or modality is to be used for documentation, for deciding on a suitable time for performing the documentation etc., and for waiting on the surgical imaging system to perform the documentation. In the proposed concept, the mental load of the surgeon is reduced by using a pre-defined set (e.g., sequence) of documentation actions, which are performed autonomously by the surgical imaging system in response to the trigger, without the user (i.e., surgeon) having to perform the documentation themselves.

The system 110 is configured to obtain a trigger for executing a set of documentation actions for documenting the surgical site 10 of the surgical procedure being performed with the help of the surgical imaging system. The system is configured to execute the set of documentation actions in response to the trigger, with the set of documentation actions comprising a plurality of actions to be performed for documenting the surgical procedure. The system is configured to store a result of the execution of the set of documentation actions, e.g., using a storage device (e.g., storage device 116 of the system) or storage circuitry of the surgical imaging system.

The proposed concept centers around the set of documentation actions, which comprises a plurality of documentation actions to be performed for documenting the surgical procedure.

This set of documentation actions may be considered a set, a sequence or checklist of documentation actions to be performed by the surgical imaging device, e.g., the system 110, the imaging device 120, and optionally the robotic arm 130, to document the surgical procedure. In any case, the set of documentation actions comprises a plurality of actions to be performed, e.g., a plurality of images (and/or image sequence(s)) to be recorded, a plurality of imaging modes being used, a plurality of imaging modalities being used etc. Which documentation actions are to be performed depends on the use case at hand, e.g., on the surgical procedure being performed (e.g., neurosurgical procedure vs. ophthalmic procedure), on the expectations and guidelines of the clinic in which the surgical procedure is being performed, on the personal preferences of the surgeon etc.

In many cases, the set of documentation actions comprises actions related to recording a plurality of images. For example, surgical imaging systems, such as surgical microscopes, offer different imaging modes. Typically, an imaging mode is a set of settings (e.g., with respect to one or more of a filter being used, an illumination intensity, an illumination spectrum, a spectrum being included in the resulting image, a sensitivity of the optical imaging sensor, a visualization parameter). Each imaging mode provides different information, e.g., a white light image captures the anatomy, ICG (indocyanine green) fluorescence provides the blood flow, and 5-ALA (5-aminolevulinic acid) fluorescence reveals the tumor. For example, the set of documentation actions may comprise an action comprising recording one or more images of the surgical site using a first imaging mode (e.g., a white light imaging mode) and a further action comprising recording one or more images of the surgical site using a second imaging mode (e.g., a fluorescence imaging mode). For example, the set of documentation actions may comprise another action comprising recording one or more images of the surgical site using a third imaging mode (e.g., a second fluorescence imaging mode), depending on how many fluorophores are being used. Depending on the imaging mode, the illumination spectrum and intensity and the spectrum being represented may be varied when capturing the respective images using the optical imaging sensor of the imaging device. In some cases, different imaging modes may also be implemented by using different imaging modalities for the first and second imaging mode, e.g., a microscope and an overview camera, an exoscope and an endoscope etc., a microscope and an optical coherence tomography (OCT) device etc.

Another aspect that is relevant for the purpose of documentation is the field of view (FOV). In many cases, a surgeon will use a zoomed-in field of view when performing the surgical procedure, resulting in a small field of view. However, in many cases, the surgical site is larger than the small portion of it the surgeon is currently working on. Therefore, images with multiple fields of view may be included in the documentation. For example, the set of documentation actions may comprise an action comprising recording one or more images of the surgical site having a first field of view and a further action comprising recording one or more images of the surgical site having a second field of view. For example, the first field of view may be a zoomed-in field of view (i.e., a first smaller field of view), and the second field of view may be a zoomed-out field of view (i.e., a second larger field of view). For example, the second field of view may include the first field of view, i.e., the first field if view may be a portion of the second field of view. For example, the system may be configured to control the imaging device 120, the robotic arm, a motorized stand 105 or one or more mirrors (not shown) to change the field of view between recording the one or more images having the first field of view and the one or more images having the second field of view, e.g., by controlling a zoom functionality of the imaging device 120 or by changing a working distance of the imaging device 120 using the robotic arm 130, motorized stand 105 or one or more mirrors.

Another change with respect to the field of view is not based on recording zoomed-in and zoomed-out images, but rather based on recording images from different angles of observation. Occasionally, a single angle of observation cannot capture all the desired aspects. For example, a "hanging" vessel may obstruct the view on background tissue. To capture the full picture of the background, it may be useful to capture multiple images from different angles of observation with complementary fields of views. For example, the set of documentation actions may comprise an action comprising recording one or more images of the surgical site having a first angle of observation and a further action comprising recording one or more images of the surgical site having a second angle of observation, with the first angle of observation being different form the second angle of observation. For example, the one or more images of the surgical site having the first angle of observation may show the surgical site as seen by the surgeon, while the second angle of observation shows the surgical site from a different angle of observation, thereby avoiding an obstacle that partially obstructs the view on the surgical site. However, recording the surgical site from different angles of observation may not always be necessary, e.g., if the surgical site is fully observable from a single angle of observation. To determine, whether multiple angles of observation are to be used, image processing may be used. For example, the system may be configured to determine (a presence of) one or more obstacles that at least partially block the view on the surgical site. This can be done in multiple ways. For example, the system may be configured to determine presence of an obstacle if, in an inner portion of the field of view (e.g., covering at least 30% and at most 80% of the field of view by area), a portion of the field of view is out of focus, indicating presence of tissue that blocks the view of the surgeon on the focal plane that the surgeon is operating on. Additionally, or alternatively, the system may be configured to generate a three-dimensional representation of the surgical site being operated on based on the captured images, and to determine whether a portion of the surgical site (e.g., an anatomical feature) or a tool is/are obstacle(s) partially obstructing the view on th surgical site based on the three-dimensional representation of the surgical site. More details on the generation of the three-dimensional representation are discussed at a later stage. If such obstacle(s) exist, images may be recorded of the surgical site from the different angles of observation, and/or the first angle of observation and/or the second angle of observation may be chosen such that the obstacle is avoided in at least one of the angles of observation. Thus, the system may be configured to determine at least one of the first angle of observation and the second angle of observation based on the one or more obstacles.

To implement the recording from different angles of observation, in general, surgical personnel may be guided to arrange the imaging device such that the different angles of observation are reached. However, more comfortably, faster, and with a higher precision, this can be done using the robotic arm 130, the motorized stand 105, or one or more mirrors of the surgical imaging device. In other words, the system may be configured to control at least one mechanical element of the surgical imaging system, such as the robotic arm 130, the motorized stand 105, or one or more mirrors, between the recording of the one or more images of the surgical site having the first angle of observation and the recording of the one or more images of the surgical site having the second angle of observation, to arrange the imaging device such that the first angle of observation and the second angle of observation are achieved.

Another aspect that is relevant for documentation is the documentation of the surgical site at different focal planes. In general, during surgery, to increase the amount of usable light, the imaging device may be used with a large aperture, which results in a shallow depth of field. While this shallow depth of field is acceptable for the surgeon during the surgical procedure, it also means that portions of the field of view outside the resulting focal plane / depth of field are shown out of focus. Therefore, when documenting the surgical procedure, images may be recorded at different focal planes, which may improve the visibility of features of the surgical site outside the depth of field of the focal plane being used by the surgeon. In other words, the set of documentation actions may comprise an action comprising recording one or more images of the surgical site having a first focal plane and a further action comprising recording one or more images of the surgical site having a second focal plane (being different from the first focal plane). For example, the first focal plane may be a focal plane being used by the surgeon during the surgical procedure, and the second focal plane may be a focal plane that is closer to the objective of the imaging device (e.g., in case the surgeon performs surgery within a wound cavity) or further away from the objective of the imaging device than the first focal plane. For example, the system may be configured to control a focussing functionality of the imaging device or the robotic arm 130 (to change the working distance, and thus the focal plane) between recording the one or more images having the first focal plane and recording the images having the second focal plane. Depending on the topography of the surgical site, images may be recorded at more than two focal planes.

In some cases, e.g., with respect to blood flow or tissue movement, still images may only provide a limited insight. In these cases, short sequences of images (e.g., a video)may be recorded (e.g., in addition to the still images) to improve the documentation. For example, the set of documentation actions may comprise at least one action comprising recording a sequence of images of the surgical site, with the sequence of images having a pre-defined length. For example, the pre-defined length may be chosen such that repetitive occurrences, such as blood flowing through blood vessels, or tissue moving due to breathing of the patient, are visualized, without causing delays due to the recording of the sequence of images (which may block recording image(s) using other imaging modalities). For example, the pre-defined length may be at most 30 seconds (or at most 15 seconds, or at most 10 seconds, or at most 5 seconds). For example, the sequence of images may be recorded using a suitable imaging mode for visualizing the repetitive occurrence being documented, e.g., using an ICG fluorescence imaging model for visualizing the flow of blood, or using white light imaging for visualizing breathing motion etc.

The execution or performance of the set of documentation actions is started in response to a trigger that is obtained by the system. In particular, the system 110 is configured to obtain a trigger for executing a set of documentation actions for documenting the surgical site 10 of the surgical procedure being performed with the help of the surgical imaging system. In a straight-forward implementation, this trigger may be a manual trigger being based on an input of the surgeon (or of a surgical assistant). For example, the system may be configured to obtain the trigger from a user of the surgical imaging system via an input device 125 of the surgical imaging system. In Fig. 1b, a button 125 located at the handles of the imaging device 120 is shown as example input device. However, other types of input devices are possible, such as a foot switch, a mouth switch, a touchscreen, a button, a voice recognition system, or a gesture recognition system. In any cases, the input may be provided explicitly by the user (e.g., the surgeon or the surgical assistant).

Alternatively (or additionally), a trigger for performing the set of documentation actions may be generated by the system itself. For example, in a simple implementation, the execution or performance of the set of documentation actions may be triggered according to a schedule, e.g., based on a timer. In other words, the system may be configured to generate the trigger according to a timer. For example, the system may be configured to generate the trigger every 10 minutes, or every 15 minutes, or every 30 minutes, or every hour.

In a more computationally advanced implementation, the system may generate the trigger based on the context provided by the surgical procedure itself. In other words, the system may identify points during the surgical procedure that are particularly good points for performing documentation, e.g., as a natural break is caused by the change of tools being used, or as some stage of the surgical procedure is completed by the surgeon, who can now relax for a short time before proceeding with the next stage of the surgical procedure. For example, the system may be configured to process imaging sensor data of the surgical imaging device 120 of the surgical imaging system to determine a progress of the surgical procedure and generate the trigger according to the progress of the surgical procedure. For example, the system may be configured to track the progress of the surgical procedure, e.g., based on a pre-operative plan. For example, the progress of the surgical procedure may comprise a plurality of stages, and the system may be configured to determine the current stage of the surgical procedure. To determine the current stage of the surgical procedure, the system may be configured to use object detection (e.g., in combination with a pre-operative plan). For example, the system may be configured to perform object detection (e.g., to detect usage/presence of one or more surgical tools, e.g., using an appropriately trained machine-learning model) on the imaging sensor data of the surgical imaging device, and determine the current stage of the surgical procedure based on the detected surgical tool(s) (and based on information on which surgical tool is used during which stage of the surgical procedure). Alternatively, the system may be configured to obtain an input from a user of the surgical imaging system (e.g., via a touchscreen or via voice recognition), with the input indicating the current stage, and therefore the progress, of the surgical procedure. When the system determines that a stage of the surgical procedure is completed (e.g., when the surgeon removes a surgical tool from the surgical site, indicating the completion of the current stage), the trigger may be generated. As the current stage of the surgical procedure is closely related to the activity of the surgeon, the system may track the activity of the surgeon for tracking the progress of the surgical procedure. In other words, the system may be configured to process the imaging sensor data of a surgical imaging device 120 of the surgical imaging system to determine an activity of a surgeon at a surgical site during the surgical procedure, and to generate the trigger in response to the surgeon removing a surgical tool from the surgical site (thus indicating the end of the stage of the surgical procedure).

Another suitable point during the surgical procedure, at which documentation can be performed, is the point when the surgeon switches between using different imaging modalities (e.g., when the surgeon switches between using an OCT device and using the microscope/exoscope, or when the surgeon switches between using an endoscope and using the microscope/exoscope) of between different imaging modes. In general, when the surgeon switches between different imaging modalities (i.e., different imaging devices), the surgeon expects some form of delay. Moreover, when switching between different imaging modalities, at both sides of the switch, one of the imaging modalities is unused, and can thus be used for documentation purposes without causing an interruption for the surgeon. Similarly, when switching between different imaging modes, the surgeon expects some delay, and two different imaging modes are used in short successions, which provides an opportunity for recording near-simultaneous images using two different imaging modes. Accordingly, the system may be configured to detect a switch between different imaging modalities or imaging modes during the surgical procedure, and to generate the trigger based on the switch between the different imaging modalities or imaging modes. For example, as a result, images may be recorded using the imaging modality or imaging mode being used (just) before the switch and the imaging modality or imaging mode being used (just) after the switch.

It is evident that the trigger being based on a manual and explicit action by the user demonstrates both the will of the user/surgeon to perform the documentation actions and the indication that the time the trigger is received is a suitable time for performing the documentation.

When the surgeon/user triggers the set of documentation action, the surgeon accepts the necessary drawbacks (i.e., the unavailability of the surgical imaging device) for the time being. When the trigger is generated automatically by the system, this may not always be the case, in particular when the documentation actions are triggered based on a timer. Therefore, in some examples, different sets of documentation actions may be defined, with one of the sets being more intrusive (i.e., causing unavailability of the surgical imaging device) and the other one being less intrusive (e.g., causing only a short black-out of the digital view). For example, the system may be configured to obtain a first trigger and a second trigger, and to execute a first set of documentation actions in response to the first trigger and a second set of documentation actions in response to the second trigger. For example, the first trigger may be a trigger that is manually and explicitly issued by the user/surgeon, and the second trigger may be a trigger that is automatically and programmatically generated (by the system). For example, the system may be configured to obtain the first trigger from a user via an input device 125 of the surgical imaging system, and to obtain the second trigger by generating the trigger based on at least one of a progress of the surgical procedure, an activity of a surgeon at a surgical site, a switch between imaging modalities, and a timer. For example, the first set of documentation actions being triggered by the first trigger may comprise a larger number of documentation actions and/or documentation actions that cause unavailability of the surgical imaging device (e.g., as the surgical imaging device is repositioned to obtain different angles of observation), and the second set of documentation actions being triggered by the second trigger may comprise a lower number of documentation and/or (only) documentation actions that cause only a short-term (e.g., less than 5 seconds, or less than 2 seconds, or less than one second) interruption in the use of the surgical imaging device.

In general, storing the result of the documentation actions may store the result of an execution/performance of the set of documentation action together using a storage device or storage circuitry. For example, the result of the execution of a set of execution actions may be stored with an annotation of the result being part of the same execution of the set of execution actions. For example, the result of a set of documentation actions may document the surgical procedure at a certain stage of the surgical procedure. For example, the result of a set of documentation actions may comprise image(s) having been recorded within a time-span defined by a start and a termination of the execution/performance of the set of documentation actions. For example, the result of a set of documentation actions may comprise image(s) having been recorded within a time-span of at most 5 minutes (or at most 2 minutes, or at most 1 minute, or at most 30 seconds, or at most 10 seconds).

In some cases, the result of different documentation actions may be combined and stored as part of the result. For example, the system may be configured to generate a combined representation of the result of at least two different documentation actions. For example, the system may be configured to generate a pseudo-color overlay of fluorescence emissions based on image(s) taken using one or more fluorescence imaging modes, combine a white-light image of the surgical site with the pseudo-color overlay of the fluorescence emissions, and to store the combination as part of the result. Additionally, or alternatively, the system may be configured to generate an image having a larger depth of field based on a plurality of images having been recorded using different focal planes (using a focus stacking technique), and to store the image as part of the result. Additionally, or alternatively, the system may be configured to stitch together multiple images to generate a collage of images. In other words, the system may be configured to, using the result of the execution of the set of documentation actions, generate a collage of a plurality of images of a surgical site being operated on, with the collage having a higher resolution than individual images of the plurality of images. The collage may then be stored as part of the result as well. For example, the collage may be generated based on images having at least partially different fields of view.

In some cases, it is useful to track the progress of the surgical procedure visually. For example, the system may be configured to, for at least one documentation action of the set of documentation actions, generate a visual representation of a difference between the results of successive executions of the documentation action (e.g., an animation, or an image highlighting the difference). For example, the system may be configured to ensure that the images being used to generate the visual representation of the difference have the same field of view, e.g., by determining that the images have the same field of view, or by cropping some of the image(s). Then, the system may generate the visual representation. For example, the system may be configured to generate an animation by creating a video/sequence of images that includes multiple still images or sequences of images of the same field of view, e.g., with a transition between the images. For example, the system may be configured to generate the image highlighting the difference using a computer vision library, e.g., by computing the difference between the images, generating an overlay highlighting the difference, and combining the overlay with the recorded images. The resulting visual representation may be stored as part of the result.

In some examples, depending on the images having been captured, it is possible to recreate the surgical site in three dimensions using the captured images. For example, the system may be configured to, using the result of the execution of the set of documentation actions, generate a three-dimensional representation of a surgical site being operated on. For example, algorithms such as stereoscopic reconstruction (computing 3D positions of points in a scene, such as the surgical site, based on 2D images taken from slightly different angles), photo-grammetry (computing a point cloud of a scene, such as the surgical site, based on 2D images taken from different angles), or multi-view stereopsis may be used to generate the three-dimensional representation of the surgical site. The three-dimensional representation of the surgical site may then be stored as part of the result.

In the proposed surgical imaging system, the optical imaging sensor is used to provide the aforementioned image(s). Accordingly, the optical imaging sensor, which may be part of the surgical imaging device 120 (e.g., of the microscope) may be configured to generate the image(s). For example, the optical imaging sensor of the surgical imaging device 120 may comprise or be an APS (Active Pixel Sensor) - or a CCD (Charge-Coupled-Device)-based imaging sensor. For example, in APS-based imaging sensors, light is recorded at each pixel using a photodetector and an active amplifier of the pixel. APS-based imaging sensors are often based on CMOS (Complementary Metal-Oxide-Semiconductor) or S-CMOS (Scientific CMOS) technology. In CCD-based imaging sensors, incoming photons are converted into electron charges at a semiconductor-oxide interface, which are subsequently moved between capacitive bins in the imaging sensors by a circuitry of the imaging sensors to perform the imaging. The system 110 may be configured to obtain (i.e., receive or read out) the image(s) from the optical imaging sensor. The image(s) may be obtained by receiving the image(s) from the optical imaging sensor (e.g., via the interface 112), by reading the image(s) out from a memory of the optical imaging sensor (e.g., via the interface 112), or by reading the image(s) from a storage device 116 of the system 110, e.g., after the image(s) has been written to the storage device 116 by the optical imaging sensor or by another system or processor.

The one or more interfaces 112 of the system 110 may correspond to one or more inputs and/or outputs for receiving and/or transmitting information, which may be in digital (bit) values according to a specified code, within a module, between modules or between modules of different entities. For example, the one or more interfaces 112 may comprise interface circuitry configured to receive and/or transmit information. The one or more processors 114 of the system 110 may be implemented using one or more processing units, one or more processing devices, any means for processing, such as a processor, a computer or a programmable hardware component being operable with accordingly adapted software. In other words, the described function of the one or more processors 114 may as well be implemented in software, which is then executed on one or more programmable hardware components. Such hardware components may comprise a general-purpose processor, a Digital Signal Processor (DSP), a micro-controller, etc. The one or more storage devices 116 of the system 110 may comprise at least one element of the group of a computer readable storage medium, such as a magnetic or optical storage medium, e.g., a hard disk drive, a flash memory, Floppy-Disk, Random Access Memory (RAM), Programmable Read Only Memory (PROM), Erasable Programmable Read Only Memory (EPROM), an Electronically Erasable Programmable Read Only Memory (EEPROM), or a network storage.

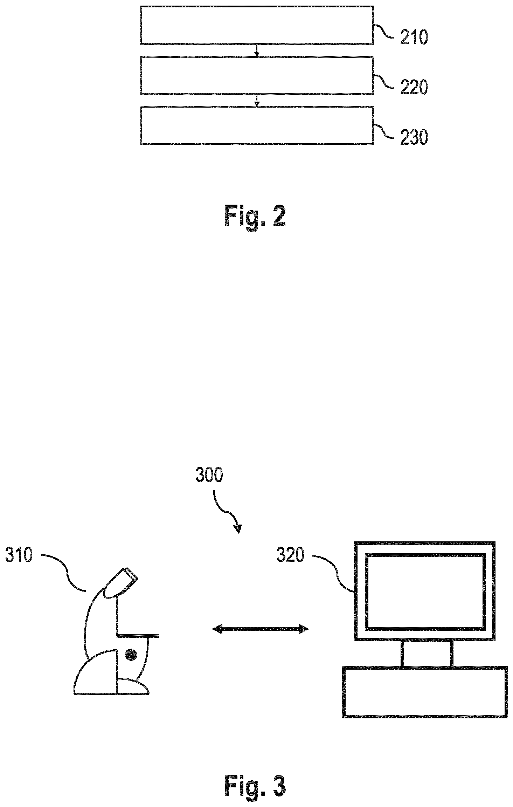

More details and aspects of the system 110 and surgical imaging system 100 are mentioned in connection with the proposed concept or one or more examples described above or below (e.g., Fig. 2 to 3). The system 110 and surgical imaging system 100 may comprise one or more additional optional features corresponding to one or more aspects of the proposed concept, or one or more examples described above or below.

Fig. 2 shows a flow chart of a corresponding method for a surgical imaging system, e.g., for the surgical imaging system 100 discussed in connection with Figs. 1a and 1b. The method comprises obtaining 210 a trigger for executing a set of documentation actions for documenting a surgical site of a surgical procedure being performed with the help of the surgical imaging system. The method comprises executing 220 the set of documentation actions in response to the trigger, the set of documentation actions comprising a plurality of actions to be performed for documenting the surgical procedure. The method comprises storing 230 a result of the execution of the set of documentation actions.

More details of the method are given with respect to the surgical imaging system 100 and system 110 of Figs. 1a and 1b. Features introduced in connection with the surgical imaging system 100 and processing system 110 may likewise be included in the corresponding method of Fig. 2.

More details and aspects of the method are mentioned in connection with the proposed concept, or one or more examples described above or below (e.g., Fig. 1a, 1b, 3). The method may comprise one or more additional optional features corresponding to one or more aspects of the proposed concept, or one or more examples described above or below.

Various examples of the present disclosure relate to an automated process of capturing multiple different data. For example, examples may relate to a documentation automation.

Medical optical imaging devices (i.e., surgical imaging devices), such as microscopes and endoscopes are often used for the documentation of operations. This often requires the capture of multiple images with different imaging modes, e.g., white light and fluorescence, and different imaging settings, such as field of view (FOV). By capturing images using different imaging modes and imaging settings, the aim is to capture with still images the experience provided by the microscope during the operation. In the present disclosure, an automated process of capturing multiple different data (e.g., images, sequences of images) to create a more complete recording of the surgical cavity is described.

A key component of the present disclosure is an automated documentation process, which captures a preprogrammed sequence of data acquisition (i.e., a set of documentation actions) with different settings. This allows capturing multiple aspects of the surgical cavity instead of a single still image or video clip. In the following, some examples are given for the use of multiple documentation recordings at one time point. For example, different imaging modes may be used for documenting the surgical procedure. Typically, an imaging mode is a set of settings (filters, illumination intensity, camera sensitivity, visualization parameters). Each imaging mode provides different information, e.g., white light image captures the anatomy, ICG fluorescence provides the blood flow, and 5-ALA fluorescence reveals the tumor. A complete documentation would require multiple recordings with each of the modes. For example, different fields of view (FOV) may be used for documenting the surgical procedure. In addition to the primary image capture, it is beneficial to capture a wider FOV image to provide an overview of the surgical cavity for better context, as zoomed in captures would concentrate on important spots with higher resolution. For example, different focal points or focal planes may be used for documenting the surgical procedure. In deeper surgical cavities, it is common that not the whole FOV is in focus. Additional images with different focus add clarification for areas out of focus at the first/main image. For example, multiple observation angles may be used for documenting the surgical procedure. It is not rare that a single observation angle cannot capture all the desired aspects. For example, a "hanging" vessel may obstruct the view to the background tissue. To capture the full picture of the background, it may be necessary to capture multiple images from different observation angles with complementary views. For example, a robotic-motorized arm may be used to automatically record the surgical site from different angles.

The proposed concept may result in a simpler and faster process. As a result, documentation may be performed more often, and, due to the standardized process (e.g., with a reproducible and accurate setting, e.g., regarding an observation angle), a comparison between different surgical stages may be facilitated. A complete set of image capturing may enable the ability to calculate additional insights. For example, multiple angle capturing may be used to create a 3D model of the tissue, and aligned images of multiple modes allow to have "multi-modal" data.

In some examples, a comparison function between different time points may be provided. In some examples, an automated merge of multi-mode data may be performed, e.g., by overlaying multiple fluorescence signals on a single image. In some examples, an automated 3D reconstruction of the surgical cavity may be performed. In some examples, an automated avoidance of obstacles to provide unobstructed view of the tissue may be performed. In some examples, a higher resolution image may be generated by combining a collage of images.

More details and aspects of the automated process of capturing multiple different data are mentioned in connection with the proposed concept, or one or more examples described above or below (e.g., Fig. 1a to 3). The automated process of capturing multiple different data may comprise one or more additional optional features corresponding to one or more aspects of the proposed concept, or one or more examples described above or below.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Some embodiments relate to an imaging device, such as a microscope or exoscope, comprising a system as described in connection with one or more of the Figs. 1a to 2. Alternatively, an imagign device may be part of or connected to a system as described in connection with one or more of the Figs. 1a to 2. Fig. 3 shows a schematic illustration of a system 300 configured to perform a method described herein. The system 300 comprises an imaging device 310 and a computer system 320. The imaging device 310 is configured to take images and is connected to the computer system 320. The computer system 320 is configured to execute at least a part of a method described herein. The computer system 320 may be configured to execute a machine learning algorithm. The computer system 320 and microscope 310 may be separate entities but can also be integrated together in one common housing. The computer system 320 may be part of a central processing system of the imaging device 310 and/or the computer system 320 may be part of a subcomponent of the imaging device 310, such as a sensor, an actor, a camera or an illumination unit, etc. of the imaging device 310.

The computer system 320 may be a local computer device (e.g., personal computer, laptop, tablet computer or mobile phone) with one or more processors and one or more storage devices or may be a distributed computer system (e.g., a cloud computing system with one or more processors and one or more storage devices distributed at various locations, for example, at a local client and/or one or more remote server farms and/or data centers). The computer system 320 may comprise any circuit or combination of circuits. In one embodiment, the computer system 320 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g., camera) or any other type of processor or processing circuit. Other types of circuits that may be included in the computer system 320 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 320 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 320 may also include a display device, one or more speakers, and a keyboard and/or controller, which can include a mouse, trackball, touch screen, voice-recognition device, or any other device that permits a system user to input information into and receive information from the computer system 320.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

Embodiments may be based on using a machine-learning model or machine-learning algorithm. Machine learning may refer to algorithms and statistical models that computer systems may use to perform a specific task without using explicit instructions, instead relying on models and inference. For example, in machine-learning, instead of a rule-based transformation of data, a transformation of data may be used that is inferred from an analysis of historical and/or training data. For example, the content of images may be analyzed using a machine-learning model or using a machine-learning algorithm. In order for the machine-learning model to analyze the content of an image, the machine-learning model may be trained using training images as input and training content information as output. By training the machine-learning model with a large number of training images and/or training sequences (e.g., words or sentences) and associated training content information (e.g., labels or annotations), the machine-learning model "learns" to recognize the content of the images, so the content of images that are not included in the training data can be recognized using the machine-learning model. The same principle may be used for other kinds of sensor data as well: By training a machine-learning model using training sensor data and a desired output, the machine-learning model "learns" a transformation between the sensor data and the output, which can be used to provide an output based on non-training sensor data provided to the machine-learning model. The provided data (e.g., sensor data, meta data and/or image data) may be preprocessed to obtain a feature vector, which is used as input to the machine-learning model.

Machine-learning models may be trained using training input data. The examples specified above use a training method called "supervised learning". In supervised learning, the machine-learning model is trained using a plurality of training samples, wherein each sample may comprise a plurality of input data values, and a plurality of desired output values, i.e., each training sample is associated with a desired output value. By specifying both training samples and desired output values, the machine-learning model "learns" which output value to provide based on an input sample that is similar to the samples provided during the training.

Apart from supervised learning, semi-supervised learning may be used. In semi-supervised learning, some of the training samples lack a corresponding desired output value. Supervised learning may be based on a supervised learning algorithm (e.g., a classification algorithm, a regression algorithm or a similarity learning algorithm. Classification algorithms may be used when the outputs are restricted to a limited set of values (categorical variables), i.e., the input is classified to one of the limited set of values. Regression algorithms may be used when the outputs may have any numerical value (within a range). Similarity learning algorithms may be similar to both classification and regression algorithms but are based on learning from examples using a similarity function that measures how similar or related two objects are. Apart from supervised or semi-supervised learning, unsupervised learning may be used to train the machine-learning model. In unsupervised learning, (only) input data might be supplied and an unsupervised learning algorithm may be used to find structure in the input data (e.g., by grouping or clustering the input data, finding commonalities in the data). Clustering is the assignment of input data comprising a plurality of input values into subsets (clusters) so that input values within the same cluster are similar according to one or more (pre-defined) similarity criteria, while being dissimilar to input values that are included in other clusters.

Reinforcement learning is a third group of machine-learning algorithms. In other words, reinforcement learning may be used to train the machine-learning model. In reinforcement learning, one or more software actors (called "software agents") are trained to take actions in an environment. Based on the taken actions, a reward is calculated. Reinforcement learning is based on training the one or more software agents to choose the actions such that the cumulative reward is increased, leading to software agents that become better at the task they are given (as evidenced by increasing rewards).

Furthermore, some techniques may be applied to some of the machine-learning algorithms. For example, feature learning may be used. In other words, the machine-learning model may at least partially be trained using feature learning, and/or the machine-learning algorithm may comprise a feature learning component. Feature learning algorithms, which may be called representation learning algorithms, may preserve the information in their input but also transform it in a way that makes it useful, often as a pre-processing step before performing classification or predictions. Feature learning may be based on principal components analysis or cluster analysis, for example.

In some examples, anomaly detection (i.e., outlier detection) may be used, which is aimed at providing an identification of input values that raise suspicions by differing significantly from the majority of input or training data. In other words, the machine-learning model may at least partially be trained using anomaly detection, and/or the machine-learning algorithm may comprise an anomaly detection component.

In some examples, the machine-learning algorithm may use a decision tree as a predictive model. In other words, the machine-learning model may be based on a decision tree. In a decision tree, observations about an item (e.g., a set of input values) may be represented by the branches of the decision tree, and an output value corresponding to the item may be represented by the leaves of the decision tree. Decision trees may support both discrete values and continuous values as output values. If discrete values are used, the decision tree may be denoted a classification tree, if continuous values are used, the decision tree may be denoted a regression tree.

Association rules are a further technique that may be used in machine-learning algorithms. In other words, the machine-learning model may be based on one or more association rules. Association rules are created by identifying relationships between variables in large amounts of data. The machine-learning algorithm may identify and/or utilize one or more relational rules that represent the knowledge that is derived from the data. The rules may e.g., be used to store, manipulate or apply the knowledge.

Machine-learning algorithms are usually based on a machine-learning model. In other words, the term "machine-learning algorithm" may denote a set of instructions that may be used to create, train or use a machine-learning model. The term "machine-learning model" may denote a data structure and/or set of rules that represents the learned knowledge (e.g., based on the training performed by the machine-learning algorithm). In embodiments, the usage of a machine-learning algorithm may imply the usage of an underlying machine-learning model (or of a plurality of underlying machine-learning models). The usage of a machine-learning model may imply that the machine-learning model and/or the data structure/set of rules that is the machine-learning model is trained by a machine-learning algorithm.

For example, the machine-learning model may be an artificial neural network (ANN). ANNs are systems that are inspired by biological neural networks, such as can be found in a retina or a brain. ANNs comprise a plurality of interconnected nodes and a plurality of connections, so-called edges, between the nodes. There are usually three types of nodes, input nodes that receiving input values, hidden nodes that are (only) connected to other nodes, and output nodes that provide output values. Each node may represent an artificial neuron. Each edge may transmit information, from one node to another. The output of a node may be defined as a (non-linear) function of its inputs (e.g., of the sum of its inputs). The inputs of a node may be used in the function based on a "weight" of the edge or of the node that provides the input. The weight of nodes and/or of edges may be adjusted in the learning process. In other words, the training of an artificial neural network may comprise adjusting the weights of the nodes and/or edges of the artificial neural network, i.e., to achieve a desired output for a given input.

Alternatively, the machine-learning model may be a support vector machine, a random forest model or a gradient boosting model. Support vector machines (i.e., support vector networks) are supervised learning models with associated learning algorithms that may be used to analyze data (e.g., in classification or regression analysis). Support vector machines may be trained by providing an input with a plurality of training input values that belong to one of two categories. The support vector machine may be trained to assign a new input value to one of the two categories. Alternatively, the machine-learning model may be a Bayesian network, which is a probabilistic directed acyclic graphical model. A Bayesian network may represent a set of random variables and their conditional dependencies using a directed acyclic graph. Alternatively, the machine-learning model may be based on a genetic algorithm, which is a search algorithm and heuristic technique that mimics the process of natural selection.

### List of reference Signs

- 100: Surgical imaging system / surgical microscope system
- 110: System
- 112: One or more interfaces
- 114: One or more processors
- 116: One or more storage devices
- 120: Surgical imaging device
- 125: Input device
- 130: Robotic arm
- 140a: Ocular displays
- 140b: Auxiliary display
- 210: Obtaining a trigger
- 220: Executing a set of documentation actions
- 230: Storing a result
- 300: System
- 310: Imaging device
- 320: Computer system

## Claims

1. A system (110) for a surgical imaging system (100), the system comprising one or more processors (114) and one or more storage devices (116), wherein the system (110) is configured to:
obtain a trigger for executing a set of documentation actions for documenting a surgical site (10) of a surgical procedure being performed with the help of the surgical imaging system (100);
execute the set of documentation actions in response to the trigger, the set of documentation actions comprising a plurality of actions to be performed for documenting the surgical procedure; and
store a result of the execution of the set of documentation actions.

2. The system (110) according to claim 1, wherein the set of documentation actions comprises an action comprising recording one or more images of the surgical site using a first imaging mode and a further action comprising recording one or more images of the surgical site using a second imaging mode.

3. The system (110) according to one of the claims 1 or 2, wherein the set of documentation actions comprises an action comprising recording one or more images of the surgical site having a first field of view and a further action comprising recording one or more images of the surgical site having a second field of view.

4. The system (110) according to one of the claims 1 to 3, wherein the set of documentation actions comprises an action comprising recording one or more images of the surgical site having a first focal plane and a further action comprising recording one or more images of the surgical site having a second focal plane.

5. The system (110) according to one of the claims 1 to 4, wherein the set of documentation actions comprises an action comprising recording one or more images of the surgical site having a first angle of observation and a further action comprising recording one or more images of the surgical site having a second angle of observation.

6. The system (110) according to claim 5, wherein the system (110) is configured to control at least one mechanical element of the surgical imaging system (100), such as a robotic arm (130), a motorized stand or one or more mirrors, between the recording of the one or more images of the surgical site having the first angle of observation and the recording of the one or more images of the surgical site having the second angle of observation.

7. The system (110) according to one of the claims 5 or 6, wherein the system (110) is configured to determine one or more obstacles that at least partially block the view on the surgical site, and to determine at least one of the first angle of observation and the second angle of observation based on the one or more obstacles.

8. The system (110) according to one of the claims 1 to 7, wherein the set of documentation actions comprises at least one action comprising recording a sequence of images of the surgical site, with the sequence of images having a pre-defined length.

9. The system (110) according to one of the claims 1 to 8, wherein the system (110) is configured to obtain the trigger from a user of the surgical imaging system (100) via an input device (125) of the surgical imaging system (100).

10. The system (110) according to one of the claims 1 to 9, wherein the system (110) is configured to process imaging sensor data of a surgical imaging device (120) of the surgical imaging system (100) to determine a progress of the surgical procedure, and to generate the trigger according to the progress of the surgical procedure,
and/or wherein the system (110) is configured to process the imaging sensor data of a surgical imaging device (120) of the surgical imaging system (100) to determine an activity of a surgeon at a surgical site during the surgical procedure, and to generate the trigger in response to the surgeon removing a surgical tool from the surgical site.

11. The system (110) according to one of the claims 1 to 10, wherein the system (110) is configured to detect a switch between different imaging modalities during the surgical procedure, and to generate the trigger based on the switch between the different imaging modalities.

12. The system (110) according to one of the claims 1 to 11, wherein the system (110) is configured to obtain a first trigger and a second trigger, and to execute a first set of documentation actions in response to the first trigger and a second set of documentation actions in response to the second trigger.

13. The system (110) according to one of the claims 1 to 12, wherein the system (110) is configured to generate a combined representation of the result of at least two different documentation actions,
and/or wherein the system (110) is configured to, for at least one documentation action of the set of documentation actions, generate a visual representation of a difference between the results of successive executions of the documentation action,
and/or wherein the system (110) is configured to, using the result of the execution of the set of documentation actions, generate a three-dimensional representation of a surgical site being operated on,
and/or wherein the system (110) is configured to, using the result of the execution of the set of documentation actions, generate a collage of a plurality of images of a surgical site being operated on, with the collage having a higher resolution than individual images of the plurality of images.

14. A method for a surgical imaging system, the method comprising:
obtaining (210) a trigger for executing a set of documentation actions for documenting a surgical site of a surgical procedure being performed with the help of the surgical imaging system;
executing (220) the set of documentation actions in response to the trigger, the set of documentation actions comprising a plurality of actions to be performed for documenting the surgical procedure; and
storing (230) a result of the execution of the set of documentation actions.

15. Computer program with a program code for performing the method according to claim 14 when the computer program is run on a processor.
